# EUROPÄISCHE PATENTSCHRIFT

(11) **EP 2 090 290 B1**
(45) Veröffentlichungstag und Bekanntmachung des Hinweises auf die Patenterteilung: **04.05.2011**
(21) Anmeldenummer: 08021769.8
(22) Anmeldetag: 16.12.2008
(51) Int. Cl.: A61K 8/35, A61Q 19/06

(54) **Verwendung von 3-(4-Hydroxy-3-methoxphenyl)-1-(4-hydroxyphenyl)-propan-1-on zur verbesserten Hautkonturierung bzw. gegen Cellulite**
Use of 3-(4-hydroxy-3-methoxyphenyl)-1-(4-hydroxphenyl)-propan-1-on for improved skin contouring and countering cellulite
Utilisation du 3-(4-hydroxy-3-méthoxphényle)-1-(4-hydroxyphényle)-propane-1-one en vue d'un meilleur contour de la peau et contre la cellulite

(30) Priorität: 18.02.2008 DE 102008009758
(43) Veröffentlichungstag der Anmeldung: 19.08.2009
(73) Patentinhaber: Beiersdorf AG, 20245 Hamburg (DE)
(72) Erfinder: Siegner, Ralf, 25421 Pinneberg (DE); Holtzmann, Ursula, 22309 Hamburg (DE); Eckert, Julia, 22085 Hamburg (DE); Heuser, Stefan, Dr., 20251 Hamburg (DE); Winnefeld, Marc, Dr., 20253 Hamburg (DE)
(74) Vertreter: Wilke, Jochen

(56) Entgegenhaltungen:
- WO-A-2007/107596
- WO-A-2007/122421
- FR-A- 2 578 165

## Beschreibung

Die vorliegende Erfindung betrifft die kosmetische oder dermatologische Verwendung von 3-(4-Hydroxy-3-methoxyphenyl)-1-(4-hydroxyphenyl)-propan-1-on zur verbesserten Hautkonturierung bzw. gegen Cellulite.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönem, um auf sichtbare, fühlbare und riechbare Weise sowohl den Mitmenschen als auch sich selbst zu gefallen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).
   Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie der unter a), e) und g) aufgeführten Phänomene.

Produkte zur Pflege erschlaffter, insbesondere gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Daüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Oftmals wird mit der erschlafften Haut auch eine Begleiterscheinung der Übergewichtigkeit und/oder der damit häufig einhergehenden sogenannten Cellullite verbunden.

Das Körperbewußtsein der Verbraucher ist in den vergangenen Jahren deutlich gestiegen. Dabei werden neben reinigenden und pflegenden Anwendungen auch zunehmend Maßnahmen ergriffen, um die Körpersilhouette zu verbessern. Die Cellulite - ein weit verbreitetes Phänomen - nimmt dabei eine zentrale Stellung ein. Das sichtbare Bild der Cellulite beruht auf einer Zunahme von Fettpolstern in der Subcutis (Unterhautfettgewebe), einer Bindegewebsschwäche sowie einer Minderung der Durchströmungsverhältnisse in den Blut- und Lymphbahnen. Die Ursache ist somit eine zum Teil anlagebedingte Schwächung des Bindegewebes mit gleichzeitigem Auftreten von vergrößerten Fettzellkammern infolge von Übergewicht, unausgewogener Ernährung, Bewegungsmangel. Die Entstehung der Cellulite kann ferner auf eine erhöhte Durchlässigkeit der Haargefäßwände zurückgeführt werden, die das Eindringen von Wasser ins Bindegewebe erlaubt.

Daneben kann an den betroffenen Hautbereichen ein lokaler Mangel an Testosteron bestehen. Jedenfalls ist Cellulite eine Erscheinung die fast niemals bei Männern zu beobachten ist.

Dehnungsstreifen (*Striae*), sind sichtbare Erscheinungen in der Unterhaut (Subkutis), die durch starke Dehnung des Gewebes, beispielsweise während der Schwangerschaft (*Striae gravidarum*) oder bei schneller Gewichtszunahme, bzw. bei Übergewicht auftreten können. Die Färbung wird durch durchscheinende Blutgefäße hervorgerufen. Die Streifen treten bevorzugt an besonders stark belasteten Geweben wie Bauch, Hüften, Gesäß, Oberarmen und Brüsten auf.

FR-A-2 578 165 und WO-A-2007/122421 offenbaren kosmetische Zubereitungen zur Pflege der Haut, die als Anti-Cellulite wirksam beschrieben werden. Diese Zubereitungen enthalten das Chalcon Hesperidin Methylchalcon.

WO-A-2007/107596 offenbart die Verwendung von 3-(4-Hydroxy-3-methoxyphenyl)-1-(4-hydroxyphenyl)-propan-1-on als Süßungsmittel.

Aufgabe der vorliegenden Erfindung war es somit, Wege zu finden, die die Nachteile des Standes der Technik vermeiden. Insbesondere war es eine Aufgabe der vorliegenden Erfindung, Zubereitungen zur Verfügung zu stellen, die eine vorteilhafte Straffung erschlaffter Haut bewirken können.

Eine weitere Aufgabe der vorliegenden Erfindung war es, hautpflegende Zubereitungen und Zubereitungen zur Pflege der Haut zur Verfügung zu stellen, wobei die Zubereitungen das Körperäußere pflegen und verschönern sollten. Dabei sollten die Zubereitungen insbesondere auch die Hautfeuchtigkeit verbessern.

Es hat sich überraschenderweise herausgestellt, daß eine kosmetische Zubereitung, dadurch **gekennzeichnet, daß** sie eine kosmetisch wirksame Menge an 3-(4-Hydroxy-3-methoxyphenyl)-1-(4-hydroxyphenyl)-propan-1-on enthält, den Nachteilen des Standes der Technik abhilft.

Die Verwendung erfindungsgemäßer Zubereitungen mit einem kosmetisch wirksamen Gehalt an 3-(4-Hydroxy-3-methoxyphenyl)-1-(4-hydroxyphenyl)-propan-1-on - z. B. in Form der genannten Beispiele - führt in überraschender Weise zu einer erheblichen Verbesserung des Aussehens der Haut, insbesondere werden durch Anwenden erfindungsgemäßer Zubereitungen
- die Barriereeigenschaften der Haut erhalten oder wiederhergestellt,
- besser der Hautaustrocknung entgegengewirkt sowie
- die Haut besser vor Umwelteinflüssen geschützt.

Durch die Verwendung erfindungsgemäßer Zubereitungen wird ferner die Hautfeuchtigkeit und die Tiefenfeuchtigkeit der Haut gesteigert, wobei diese hautbefeuchtende Pflege-Wirkung und der Schutz vor Feuchtigkeitsverlust über mehrere Stunden bzw. sogar über den ganzen Tag oder noch länger anhält.

Erfindungsgemäß ist daher auch die Verwendung kosmetischer Zubereitungen, die eine kosmetisch wirksame Menge an 3-(4-Hydroxy-3-methoxyphenyl)-1-(4-hydroxyphenyl)-propan-1-on enthalten, zur Steigerung der Hautfeuchtigkeit bzw. zur Befeuchtung der Haut.

Es war ferner überraschend, dass durch Anwenden erfindungsgemäßer Zubereitungen die Elastizität der Haut, der Haare und/oder der Nägel und daher Cellulite bzw. das Erscheinungsbild der sog. "Orangenhaut" verbessert wird.

Erfindungsgemäß ist auch die Verwendung von 3-(4-Hydroxy-3-methoxyphenyl)-1-(4-hydroxyphenyl)-propan-1-on zur Steigerung der Spannkraft und Elastizität der Haut.

Erfindungsgemäß ist auch die Verwendung von 3-(4-Hydroxy-3-methoxyphenyl)-1-(4-hydroxyphenyl)-propan-1-on zur Festigung des Bindegewebes der Haut.

Erfindungsgemäß ist auch die Verwendung von 3-(4-Hydroxy-3-methoxyphenyl)-1-(4-hydroxyphenyl)-propan-1-on zur Minderung von Dehnungsstreifen der Haut.

Bevorzugt enthalten kosmetische oder dermatologische Zubereitungen gemäß der Erfindung 0,001 bis 30 Gew.-%, bevorzugt 0,1 bis 15 Gew.-%, besonders bevorzugt 0,5 bis 5 Gew.-% an 3-(4-Hydroxy-3-methoxyphenyl)-1-(4-hydroxyphenyl)-propan-1-on, bezogen auf das Gesamtgewicht der Zubereitung.

Herstellungsverfahren für das 3-(4-Hydroxy-3-methoxyphenyl)-1-(4-hydroxyphenyl)-propan-1-on 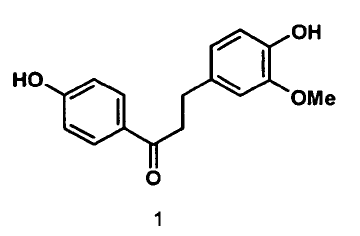 können vorteilhaft aus den folgenden Schritten bestehen oder diese umfassen:
a) Umsetzung von p-Hydroxyacetophenon mit Vanillin zu 3-(4-Hydroxy-3-methoxyphenyl)-1-(4-hydroxyphenyl)-2-propen-1-on und
b) Hydrierung des in Schritt a) gewonnenen 3-(4-Hydroxy-3-methoxyphenyl)-1-(4-hydroxyphenyl)-2-propen-1-on zu 3-(4-Hydroxy-3-methoxyphenyl)-1-(4-hydroxyphenyl)-propan-1-on.

In einer bevorzugten Ausgestaltung des erfindungsgemäßen Herstellverfahrens für die Verbindung der Formel 1 wird die Umsetzung von p-Hydroxyacetophenon mit Vanillin in Schritt a) so durchgeführt, daß p-Hydroxyacetophenon und Vanillin einer Mischung aus Kaliumhydroxid in Diethylenglykoldiethylether bei erhöhter Temperatur zugesetzt wird. Die Temperatur liegt üblicherweise im Bereich von 50 bis 200 °C, bevorzugt im Bereich von 80°C bis 140°C, weiter bevorzugt im Bereich von 100 bis 120°C.

Anschließend wird die entstandene Verbindung bevorzugt zu 3-(4-Hydroxy-3-methoxyphenyl)-1-(4-hydroxyphenyl)-2-propen-1-on hydrolysiert. Bevorzugt wird der pH-Wert im Bereich von 6 bis 7 eingestellt.

In einer bevorzugten Ausgestaltung des erfindungsgemäßen Herstellverfahrens für die Verbindung der Formel 1 wird die Hydrierung des in Schritt (a) gewonnenen 3-(4-Hydroxy-3-methoxyphenyl)-1-(4-hydroxyphenyl)-2-propen-1-on zu 3-(4-Hydroxy-3-methoxyphenyl)-1-(4-hydroxyphenyl)-propan-1-on so durchgeführt, daß das entstandene 3-(4-Hydroxy-3-methoxyphenyl)-1-(4-hydroxyphenyl)-2-propen-1-on in Tetrahydrofuran gelöst und mit einer geeigneten Menge Pd auf Aktivkohle (Pd-Gehalt: 5 Gew.-%, Wassergehalt ca. 50 Gew.-%, jeweils bezogen auf die Gesamtmasse des Katalysators) versetzt wird. Vorzugsweise wird die Reaktion bei Normaldruck und weiter vorzugsweise bei Raumtemperatur (ca. 20°C) vorgenommen.

### Synthesebeispiel für 3-(4-Hydroxy-3-methoxyphenyl)-1-(4-hydroxyphenyl)-1-propanon

### (a) Umsetzung von p-Hydroxyacetophenon mit Vanillin:

Es werden 20 g Kaliumhydroxid in 100 g Diethylenglykoldiethylether vorgelegt, auf 120°C unter Rühren erhitzt und mit einer Mischung von 14 g p-Hydroxyacetophenon und 15 g Vanillin innerhalb von 1 h versetzt. Nach beendeter Dosierung rührt man noch 20 min nach, hydrolysiert und stellt auf pH 6-7 ein. Nach Phasentrennung entfernt man das Lösungsmittel und erhält 25 g 3-(4-Hydroxy-3-methoxyphenyl)-1-(4-hydroxyphenyl)-2-propen-1-on, Ausbeute: 93% der Theorie.

### (b) Hydrierung des in Schritt (a) gewonnenen Reaktionsprodukts:

10 g 3-(4-Hydroxy-3-methoxyphenyl)-1-(4-hydroxyphenyl)-2-propen-1-on werden in 100 g Tetrahydrofuran gelöst, mit 0,2 g Pd auf Aktivkohle (Pd-Gehalt: 5 Gew.-%, Wassergehalt ca. 50 Gew.-%, jeweils bezogen auf die Gesamtmasse des Katalysators) versetzt und bei Normaldruck und Raumtemperatur (ca. 20°C) hydriert. Man erhält nach Entfernung des Katalysators und des Lösungsmittels 9 g an 3-(4-Hydroxy-3-methoxyphenyl)-1-(4-hydroxyphenyl)-propan-1-on. Ausbeute: 89% der Theorie

Spektroskopische Daten von 3-(4-Hydroxy-3-methoxyphenyl)-1-(4-hydroxyphenyl)-propan-1-on (Formel 1):
¹³C-NMR (CDCl₃; 75,5 MHz): δ (ppm) = 197,42(s), 161,85(s), 147,24(s), 144,44(s), 132,03(s), 130,37(d), 130,37(d), 128,18(s), 120,27(d), 115,11(d), 115,07(d), 115,07(d), 112,53(d), 55,42(q), 39,30(t), 29,42(t);
MS: m/z (%) = M⁺-Ion 272(82), 151(24), 137(77), 121(100), 93(19), 65(22).

Die kosmetischen oder dermatologischen Zubereitungen können erfindungsgemäß wie üblich zusammengesetzt sein und zur Behandlung, der Pflege und der Reinigung der Haut und/oder der Haare und als Schminkprodukt in der dekorativen Kosmetik dienen.

Sie enthalten bevorzugt 0,001 Gew.-% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an 3-(4-Hydroxy-3-methoxyphenyl)-1-(4-hydroxyphenyl)-propan-1-on.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen.

Es ist auch erfindungsgemäß vorteilhaft, 3-(4-Hydroxy-3-methoxyphenyl)-1-(4-hydroxyphenyl)-propan-1-on in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, 3-(4-Hydroxy-3-methoxyphenyl)-1-(4-hydroxyphenyl)-propan-1-on in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Gelbildner, Schaumstabilisatoren, Elektrolyte, organische Lösemittel oder Siliconderivate

Vorteilhafte **Gelbildner** für derartige Zubereitungen sind beispielsweise Copolymere aus C₁₀₋₃₀-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die Pemulen® Typen TR 1, TR 2 und TRZ von der Fa. Goodrich (Noveon)

Auch Carbopole sind vorteilhafte Gelbildner für derartige Zubereitungen. Carbopole sind Polymere der Acrylsäure, insbesondere auch Acrylat-Alkylacrylat-Copolymere. Vorteilhafte Carbopole sind beispielsweise die Typen 980, 981, 984 1342, 1382, 2984 und 5984. ebenso die ETD-Typen 2001, 2020, 2050 und Carbopol Ultrez 10, PVM/MA Decadien Crosspolymer (Handelsname Stabileze® 06), Polyglycerylmethacrylat sowie Polyacrylamid, Ammoniumdimethyltauramide/vinylformamidcopolymere, Copolymere oder Crosspolymere umfassend Aacryloyldimethyltaurate, Polyacryloyl-dimethyltauramide, Polyvinylpyrrolidone und Copolymere davon.

Weitere vorteilhafte Gelbildner für derartige Zubereitungen sind Xanthan Gummi, Polyvinylpyrrolidon, Cellulosederivate, insbesondere Celluloseether wie zum Beispiel Hydroxypropylmethylcellulose, Stärke und Stärkederivate, Hyaluronsäure, Carrageenan, Siliciumdioxid und Aluminiumsilikate.

Die Menge an Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Siliconöle wie Dimethicone, Cyclomethicone, Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, Gewichtsprozente, auf das Gewicht und die Gesamtmenge bzw. auf das Gesamtgewicht der Zubereitungen bezogen.

| Firming-Lotion mit 3-(4-Hydroxy-3-methoxyphenyl)-1-(4-hydroxyphenyl)-1-propanon | Gew.-% |
|---|---|
| Paraffinum Liquidum | 10 |
| Glycerylstearat SE | 1.5 |
| Stearinsäure | 1 |
| Glycerin | 10 |
| Carbomer | 0.2 |
| Xanthangummi | 1 |
| Coffein | 0.5 |
| Alcohol Denat. | 3 |
| Coenzym Q 10 | 0.03 |
| Dimethicon | 2 |
| Cyclomethicon | 3 |
| 3-(4-Hydroxy-3-methoxyphenyl)-1-(4-hydroxyphenyl)-1-propanon | 0.1 |
| Parfum | 0.2 |
| Konservierung | q.s. |
| Wasser | ad 100 |

| Anti-Cellulite Creme | Gew.-% |
|---|---|
| PEG-40 Stearat | 1.8 |
| Cetylalcohol | 3 |
| Glycerylstearat | 3 |
| Dimethicone | 2 |
| Paraffinum Liquidum | 3 |
| Glycerine | 8 |
| Carbomer | 0.2 |
| Caprylsäure-/Caprinsäuretriglyceride | 3 |
| 3-(4-Hydroxy-3-methoxyphenyl)-1-(4-hydroxyphenyl)-1-propanon | 0.5 |
| EDTA | 0.2 |
| Coffein | 2 |
| Konservierung | q.s. |
| Wasser | ad 100 |

| Anti-Cellulite Gel | Gew.-% |
|---|---|
| Natriumpolyacrylat | 0.4 |
| Chondrus Chrispus | 0.1 |
| Carbomer | 0.3 |
| Cyclomethicon | 4 |
| Glycerin | 8 |
| Dimethiconol | 2 |
| Alcohol Denat. | 10 |
| Ginkgo Biloba | 0,1 |
| Aescin | 0,05 |
| Carnitin | 0.3 |
| Coffein | 0.2 |
| 3-(4-Hydroxy-3-methoxyphenyl)-1-(4-hydroxyphenyl)-1-propanon | 0.2 |
| Wasser | ad 100 |

| Anti-Celluliteserum | Gew.-% |
|---|---|
| Acrylates/C 10-30 Alkyl Acrylate Crosspolymer | 0.5 |
| Xanthangummi | 0.2 |
| Cetearylalkohol | 1 |
| Dimeticone | 1 |
| Cyclomethicone | 5 |
| Glycerin | 4 |
| Hydrogenated Polyisobuten | 2 |
| Alcohol Denat. | 10 |
| Phenoxyethanol | 0,4 |
| Methylparaben | 0,2 |
| 3-(4-Hydroxy-3-methoxyphenyl)-1-(4-hydroxyphenyl)-1-propanon | 0.5 |
| Wasser | ad 100 |

## Patentansprüche

1. Verwendung von 3-(4-Hydroxy-3-methoxyphenyl)-1-(4-hydroxyphenyl)-propan-1-on zur Steigerung der Hautfeuchtigkeit bzw. zur Befeuchtung der Haut.

2. Verwendung von 3-(4-Hydroxy-3-methoxyphenyl)-1-(4-hydroxyphenyl)-propan-1-on zur Verbesserung der Hautoberflächenstruktur, insbesondere zur Glättung der Haut und zur Verringerung von Hautdellen.

3. Verwendung von 3-(4-Hydroxy-3-methoxyphenyl)-1-(4-hydroxyphenyl)-propan-1-on zur Verminderung von Cellulite.

4. Verwendung von 3-(4-Hydroxy-3-methoxyphenyl)-1-(4-hydroxyphenyl)-propan-1-on zur Steigerung der Spannkraft und Elastizität der Haut.

5. Verwendung von 3-(4-Hydroxy-3-methoxyphenyl)-1-(4-hydroxyphenyl)-propan-1-on zur Festigung des Bindegewebes der Haut.

6. Verwendung von 3-(4-Hydroxy-3-methoxyphenyl)-1-(4-hydroxyphenyl)-propan-1-on zur Minderung von Dehnungsstreifen der Haut.

7. Verwendung nach einem der vorhergehenden Ansprüche, **dadurch gekennzeichnet, daß** die Konzentration an 3-(4-Hydroxy-3-methoxyphenyl)-1-(4-hydroxyphenyl)-propan-1-on in kosmetischen oder dermatologischen Zubereitungen von 0,0001 bis 30 Gew.-% gewählt wird.

8. Verwendung nach einem der vorhergehenden Ansprüche, **dadurch gekennzeichnet, daß** die Konzentration an 3-(4-Hydroxy-3-methoxyphenyl)-1-(4-hydroxyphenyl)-propan-1-on in kosmetischen oder dermatologischen Zubereitungen von 0,01 bis 10 Gew.-% gewählt wird.

9. Verwendung nach einem der vorhergehenden Ansprüche, **dadurch gekennzeichnet, daß** die Konzentration an 3-(4-Hydroxy-3-methoxyphenyl)-1-(4-hydroxyphenyl)-propan-1-on in kosmetischen oder dermatologischen Zubereitungen von 0,0001 bis 30 Gew.-% gewählt wird.

10. Verwendung nach einem der vorhergehenden Ansprüche, **dadurch gekennzeichnet, daß** die Konzentration an 3-(4-Hydroxy-3-methoxyphenyl)-1-(4-hydroxyphenyl)-propan-1-on in kosmetischen oder dermatologischen Zubereitungen von 0,01 bis 10 Gew.-% gewählt wird.

## Claims

1. Use of 3-(4-hydroxy-3-methoxphenyl)-1-(4-hydroxyphenyl)propan-1-one for increasing the skin moisture and for moisturizing the skin.

2. Use of 3-(4-hydroxy-3-methoxphenyl)-1-(4-hydroxyphenyl)propan-1-one for improving the structure of the skin surface, in particular for smoothing the skin and for reducing skin dimples.

3. Use of 3-(4-hydroxy-3-methoxphenyl)-1-(4-hydroxyphenyl)propan-1-one for reducing cellulite.

4. Use of 3-(4-hydroxy-3-methoxphenyl)-1-(4-hydroxyphenyl)propan-1-one for increasing the tone and elasticity of the skin.

5. Use of 3-(4-hydroxy-3-methoxphenyl)-1-(4-hydroxyphenyl)propan-1-one for strengthening the connective tissue in the skin.

6. Use of 3-(4-hydroxy-3-methoxphenyl)-1-(4-hydroxyphenyl)prapan-1-one for reducing stretch marks in the skin.

7. Use according to one of the preceding claims, **characterized in that** the concentration of 3-(4-hydroxy-3-methoxphenyl)-1-(4-hydroxyphenyl)propan-1-one in cosmetic or dermatological preparations is chosen to be from 0.0001 to 30% by weight.

8. Use according to one of the preceding claims, **characterized in that** the concentration of 3-(4-hydroxy-3-methoxphenyl)-1-(4-hydroxyphenyl)propan-1-one in cosmetic or dermatological preparations is chosen to be from 0.01 to 10% by weight.

9. Use according to one of the preceding claims, **characterized in that** the concentration of 3-(4-hydroxy-3-methoxphenyl)-1-(4-hydroxyphenyl)propan-1-one in cosmetic or dermatological preparations is chosen to be from 0.0001 to 30% by weight.

10. Use according to one of the preceding claims, **characterized in that** the concentration of 3-(4-hydroxy-3-methoxphenyl)-1-(4-hydroxyphenyl)propan-1-one in cosmetic or dermatological preparations is chosen to be from 0.01 to 10% by weight.

## Revendications

1. Utilisation de 3-(4-hydroxy-3-méthoxyphényl)-1-(4-hydroxyphényl)-propan-1-one pour augmenter l'hydratation de la peau ou, selon le cas, pour hydrater la peau.

2. Utilisation de 3-(4-hydroxy-3-méthoxyphényl)-1-(4-hydroxyphényl)-propan-1-one pour améliorer la structure superficielle de la peau, en particulier pour lisser la peau et pour diminuer les creux dans la peau.

3. Utilisation de 3-(4-hydroxy-3-méthoxyphényl)-1-(4-hydroxyphényl)-propan-1-one pour diminuer la cellulite.

4. Utilisation de 3-(4-hydroxy-3-méthoxyphényl)-1-(4-hydroxyphényl)-propan-1-one pour augmenter la tonicité et l'élasticité de la peau.

5. Utilisation de 3-(4-hydroxy-3-méthoxyphényl)-1-(9-hydroxyphényl)-propan-1-one pour affermir le tissu conjonctif de la peau.

6. Utilisation de 3-(4-hydroxy-3-méthoxyphényl)-1-(4-hydroxyphényl)-propan-1-one pour diminuer les vergetures de la peau.

7. Utilisation selon l'une quelconque des revendications précédentes, **caractérisée en ce que** la concentration en 3-(4-hydroxy-3-méthoxyphényl)-1-(4-hydroxyphényl)-propan-1-one dans des préparations cosmétiques ou dermatologiques est choisie de 0,0001 à 30% en poids.

8. Utilisation selon l'une quelconque des revendications précédentes, **caractérisée en ce que** la concentration en 3-(4-hydroxy-3-méthoxyphényl)-1-(4-hydroxyphényl)-propan-1-one dans des préparations cosmétiques ou dermatologiques est choisie de 0,01 à 10% en poids.

9. Utilisation selon l'une quelconque des revendications précédentes, **caractérisée en ce que** la concentration en 3-(4-hydroxy-3-méthoxyphényl)-1-(4-hydroxyphényl)-propan-1-one dans des préparations cosmétiques ou dermatologiques est choisie de 0,0001 à 30% en poids.

10. Utilisation selon l'une quelconque des revendications précédentes, **caractérisée en ce que** la concentration en 3-(4-hydroxy-3-méthoxyphényl)-1-(4-hydroxyphényl)-propan-1-one dans des préparations cosmétiques ou dermatologiques est choisie de 0,01 à 10% en poids.
